# EUROPEAN PATENT APPLICATION

(11) **EP 4 623 896 A1**
(43) Date of publication of application: **01.10.2025**
(21) Application number: 23892823.8
(22) Date of filing: 22.11.2023
(51) Int. Cl.: A61K 8/99, A61Q 5/06

(54) **COSMETIC COMPOSITION FOR INDUCING MELANIN SYNTHESIS IN AN INDIVIDUAL OR ANIMAL; PRODUCTION PROCESS; COSMETIC METHOD AND USE**

(30) Priority: 23.11.2022 BR 102022023843
(71) Applicant: Chemyunion Ltda, 18087-800 Sorocaba (BR)
(72) Inventor: VIDAL MAGALHÃES, Wagner, 18090-360 Sorocaba (BR); BUENO DE CAMARGO JUNIOR, Flávio, 18013-004 Sorocaba (BR); ROBERTO ROSSAN, Marcos, 18103-185 Sorocaba (BR); THOMAZ CAMILO, Andrew, 18020-252 Sorocaba (BR)
(74) Representative: Hoffmann Eitle
(86) International application number: PCT/BR2023/050406
(87) International publication number: WO 2024/108286

(57) **Abstract**

The present invention describes a new composition for inducing melanin synthesis in an individual or animal, including its use for preventing and/or reversing the graying process of hair strands.

## Description

### Technical Field of the Invention

The present invention describes a new composition for inducing melanin synthesis in an individual or animal, including its use for preventing and/or reversing the graying process of hair strands. The present invention is in the fields of cosmetic, dermatological and/or pharmaceutical treatments.

### Background of the Invention

Hair color, like skin color, is a result of the quantity and type of melanin produced by melanocytes and transferred to keratinocytes. Pigmentation of the hair follicle involves the melanogenic activity of follicular melanocytes, the transfer of melanin granules to cortical and medullary keratinocytes, and the formation of pigmented hair shafts (SLOMINSKI et al., 2005).

Gray hair is one of the most visible indicators of aging in humans. The modulation of the graying process results from numerous factors, such as genetic, oxidative, psycho-emotional, metabolic, endocrine and nutritional (O'SULLIVAN et al., 2021).

More specifically, the appearance of white hair is a phenomenon associated with a decrease in the population of follicular melanocytes and a decrease in melanin content (SARIN; ARTANDI, 2007). As for the density of follicular melanocytes, several mechanisms interfere with this factor: the loss of melanocyte stem cells or their failure in differentiation, defects in melanocyte migration, melanocyte apoptosis and malfunction of the pigment machinery (TOBIN, 2011).

As with melanocyte density, it is essential to highlight that the modulation of melanin synthesis plays an equally important role. Melanins in the skin and hair follicles are produced inside organelles called melanosomes, found in the cytoplasm of melanocytes. Melanocytes derive from immature melanocytes. During embryogenesis, these cells migrate from the neural crest, a transient component of the ectoderm located between the neural tube and the epidermis, reach the basal layer of the epidermis and then enter the developing hair follicles (SLOMINSKI et al., 2005; TOBIN, 2008). The factors that lead to the subpopulation of these neural crest cells to become melanocytes remain poorly understood, but it is known that several factors are involved, including MITF (microphthalmia-associated transcription factor), SOX10 transcription factor, Pax3 protein, KIT, FGF-2 (fibroblast growth factor-2), endothelin 3 and others (TOBIN, 2008).

In the hair follicle, the melanin produced is transferred from the melanocytes to the keratinocytes of the hair bulb (SLOMINSKI et al., 2005). Melanin is produced during a complex pathway that involves a series of chemical and enzymatic reactions, known as melanogenesis. The color that constitutes an individual's hair is the result of the activity of the enzyme tyrosinase (TYR), so its regulation and activity are crucial during the process of melanin synthesis (TOBIN, 2008). In addition to tyrosinase, the enzymes tyrosinase-related protein 1 (TRP-1) and tyrosinase-related protein 2 (TRP-2), also known as dopachrome tautomerase (DCT), are important mediators of this process (PILLAIYAR et al., 2017).

Melanogenesis begins with the oxidation of tyrosine into dopa and from this into dopaquinone by the action of TYR in the presence of molecular oxygen. This first step is limiting in the synthesis of melanin, since the rest of the reaction can occur spontaneously at a physiological pH value (PILLAIYAR et al., 2017). From this moment on, the presence or absence of cysteine will determine which pigment, eumelanin or pheomelanin, will be formed.

In the absence of cysteine, dopaquinone is converted to leucodopachrome and then to dopachrome by auto-oxidation. Dopachrome can then, by spontaneous decarboxylation, generate 5,6-dihydroxyindole (DHI), which rapidly oxidizes and polymerizes to form insoluble, high-molecular-weight polymers of dark brown-black color, belonging to the eumelanin group. However, if TRP-2/DCT is available, dopachrome can undergo the action of this enzyme and produce 5,6-dihydroxy-2-carboxylic acid (DHICA), which, through the action of TRP-1, will also form eumelanin. In the presence of cysteine, dopaquinone reacts with this molecule, forming cysteine, which then oxidizes and polymerizes, giving rise to soluble red-yellow pigments, collectively known as pheomelanins (PILLAIYAR et al., 2017).

Melanogenesis can be regulated by several pathways. It is important to highlight that the stimulation of this process occurs mainly by the pro-opiomelanocortin peptide (POMC) in keratinocytes. After the production and subsequent cleavage of this molecule, different products are produced, including melanocyte-stimulating hormone (α-MSH), adrenocorticotropic hormone (ACTH), β-lipotropin (β-LPH), β-endorphin (β-END), as well as β-MSH and γ-MSH. ACTH and α-MSH are considered some of the main stimulators of melanogenesis (PILLAIYAR et al., 2017; SCHERER et al., 2010).

Different strategies have been developed with the aim of reversing the graying process.

Reversal of hair follicle hypopigmentation associated with some nutritional deficiencies through iron supplementation in patients with anemia has been reported (BHAT et al., 2013). Topical treatments have been marketed and may contain a variety of compounds (para-aminobenzoic acid, phytic acid, capixyl, calcium pantothenate, etc.), but results are often inconsistent and only temporary (SEHRAWAT et al., 2017). The basis of treatment is often to camouflage gray hair with dyes, with some studies showing that hair dyes protect hair against photodamage (SEHRAWAT et al., 2017). More promising treatment approaches involve anti-aging or antioxidant compounds, recombinant human growth hormone therapy, and the use of α-melanocyte-stimulating hormone (α-MSH) agonists, such as Greyverse^{™} (Glycerin (and) Water (and) Palmitoyl Tetrapeptide-20 Amide - IFF Lucas Meyer) - Patent Document AU2008271875A1. As a consequence of activating the MC1R receptor, GreyverseTM increases the synthesis of the receptor itself, indicating the central mechanism of action of the activity.

Another example of a product is the combination of Taxifolin Glucoside and Acetyl Tyrosine (Darkenyl ^{™} - Givaudan) - Patent document WO2020089216A1. According to the results presented, the product induces, in a statistically significant manner, the gene expression of, among other genes, MC1R and POMC. Another example of a commercially available topical cosmetic product intended to combat graying is EUK-134 (Ethylbisiminomethylguaiacol Manganese Chloride - Lucas Meyer Cosmetics). The latter consists of a mimetic of two natural skin enzymes, superoxide dismutase and catalase, which play an important antioxidant role and, therefore, act more precisely in combating oxidative stress, but not exactly in stimulating melanin synthesis.

Other patent documents may be cited as describing inventions intended to reverse graying.

CN102988235 describes the association of deer antler, Radix Ginseng Rubra Radix Polygoni Multiflori, Semen sojae atricolor, and Brassica campestris oil capable of promoting natural hair repigmentation.

KR102265875 describes a composition intended for darkening hair and preventing graying using exosomes derived from stem cells.

WO2014096086 describes the topical use of the microorganism *Bifidobacterium longum,* in the form of lysate, for pro-pigmenting action.

PI0916690 (WO2010013182) discloses the oral cosmetic use of a probiotic microorganism (i.e. a live microorganism) of Lactobacillus paracasei or its lysate for the cosmetic treatment of oily scalp disorders, particularly scalp dandruff.

KR102570587, in turn, describes the use of a product resulting from the fermentation of a microorganism of the genus *Lactobacillus ,* that is, a mixture obtained from the fermentation of a culture medium containing an energy source such as skimmed milk powder with *Lactobacillus* to prevent gray hair or improve gray hair. The aforementioned document at no time describes the species of microorganism tested and that generated the experimental results, contenting itself with saying that it is a culture of *Lactobacillus* selected from a total of 4,000 species (example 1). Furthermore, it generically describes that the strain may be one of Lactobacillus pentosus, Lactobacillus brevis, Lactobacillus plantarum, Lactobacillus casei and Lactobacillus acidophilus. According to the publication, the fermented product must contain a large amount of carboxyl groups, which react with the amino acid inside the hair strand to change the color of the strand through a reaction external to the fiber at room temperature. It is, therefore, not only a product of a different nature to the present invention, but also an agent that acts chemically on the thread, externally.

Furthermore, according to the search for the state of the art carried out, there are several examples of products with antimelanogenic action produced with compositions and/or extracts of *Lactobacillus,* such as those described in Lim, HY et al. "Antiwrinkle and Antimelanogenesis Effects of Tyndallized Lactobacillus acidophilus KCCM12625P ". Int. J. Mol. Sci. 2020, 21, 1620 which uses *Lactobacillus acidophilus .* Choi, W. S et al. "Whitening Effects of Lactobacillus rhamnosus Associated with Its Antioxidative Activities" Korean J. Microbiol. Biotechnol. (2013), 41(2), 183-189, which use *Lactobacillus rhamnosus* and/or *Lactobacillus casei.* Bajpai, VK et al. " Partially Purified Exo-Polysaccharide from Lactobacillus Sakei Probio 65 with Antioxidant, α - Glucosidase and Tyrosinase Inhibitory Potential" J. Food Biochemistry, 40 issue 3, (2016), p. 264-274 that uses *Lactobacillus sakei* . Kim HR, et al. "Lipoteichoic acid isolated from Lactobacillus plantarum inhibits melanogenesis in B16F10 mouse melanoma cells. Mol Cells". 2015 ;38(2): 163-70 which uses *Lactobacillus plantarum.*

This demonstrates that the technical field has a high inherent unpredictability and that products that may seem similar will not necessarily be equally effective.

Thus, this patent application aims to present new ways of solving problems of in the state of the art. From what can be inferred from the literature researched, no documents were found anticipating or suggesting the teachings of the present invention.

### Summary of the Invention

Thus, the present invention aims to solve the problems in the state of the art by means of a new composition that surprisingly has a pro-melanogenesis effect.

The composition of the present invention showed results of inhibition of POM gene expression, in addition to decreasing the expression of MC1R, both important biomarkers in the process of melanogenesis and, consequently, hair pigmentation. Furthermore, the composition of the present invention was able to positively modulate the expression of other genes (e.g. TYR, DCT, TYRP-1, MITF, MYO5A, PMEL, MLPH), suggesting a mechanism of action different from that presented in the state of the art.

In this way, a new cosmetic composition is provided for the induction of melanin synthesis in an individual or animal comprising at least 0.0001% by weight of lysate of microorganisms of the species *Lacticaseibacillus paracasei.*

In one embodiment of the invention, the composition comprises between 0.001% and 20% by weight of lysate.

In one embodiment of the invention, the composition comprises between 0.001% and 5% by weight of lysate.

In one embodiment of the invention, the composition is for preventing and/or reversing the graying process of hair strands.

In one embodiment of the invention, the composition is for inducing melanin synthesis in hair follicles.

In one embodiment of the invention, the composition is a topical hair cosmetic composition.

A new process for producing a composition as defined above is also presented, comprising:
- anaerobic cultivation of microorganisms of the species *Lacticaseibacillus paracasei* in culture medium until the stationary phase;
- total or partial inactivation and lysis of microorganisms by subjecting the medium to a temperature between 55°C and 80°C for at least 30 minutes;
- filtration and centrifugation;
- mixing the resulting lysate with at least one suitable cosmetic vehicle.

A cosmetic method of inducing melanin synthesis in an individual or animal to prevent and/or reverse the graying process of hair strands is also presented, comprising the topical administration to the hair follicles of a composition as previously described, in a cosmetically suitable quantity to allow said induction in an individual or animal.

The use of the composition, as defined above, for the production of a cosmetic composition for inducing melanin synthesis in an individual or animal for preventing and/or reversing the graying process of hair strands is also presented.

In one embodiment of the invention, the composition is for application to a region of skin comprising hair follicles.

These and other objects of the invention will be immediately recognizable to those skilled in the art and descriptions will be provided below in sufficient detail for their reproduction by a person skilled in the art.

### Brief Description of Figures

In order to better define and clarify the content of this patent application, the following figure is presented:

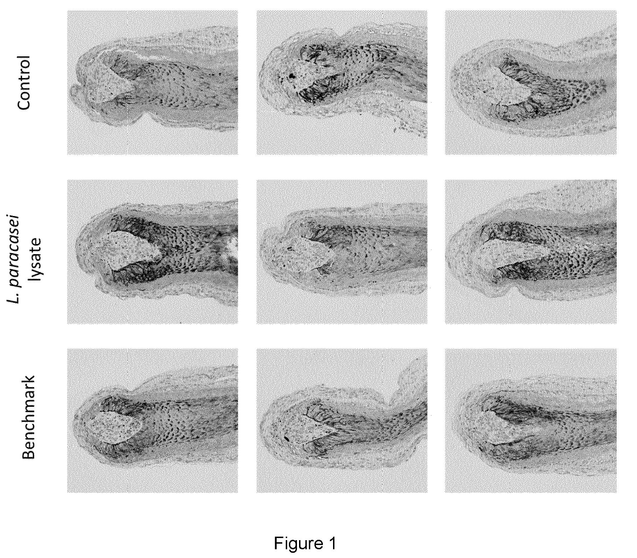

Figure 1: Representative images of the effect of *Lacticaseibacillus paracasei lysate* on melanin synthesis in *ex vivo hair follicles* - Warthin Starry staining (objective lens x40), compared to the same formulation without the addition of lysate (placebo) and a *benchmark composition .*

### Detailed Description of the Invention

This detailed description of the invention establishes some nonlimiting definitions of the main terminologies and technical characteristics used throughout this patent application, as well as providing examples of some of the embodiments of the present invention so that it can be reproduced by a person skilled in the art.

Thus, unexpectedly, the inventors observed that certain microorganisms, of *the Lacticaseibacillus paracasei type,* can be particularly effective in preventing the graying process of hair, as well as reversing its effects.

Thus, as follows from the data presented in the examples, the inventors characterized the ability of the *Lacticaseibacillus paracasei lysate* to stimulate the expression of a surprising number of genes related to melanin production in the hair follicle, in addition to directly demonstrating the stimulation of melanin production in follicle culture.

*Lacticaseibacillus paracasei* lysate was able to stimulate the gene expression of Tyrosinase (TYR), Dopachrome tautomerase (DCT), Tyrosinase-related protein-1 (TYRP-1), Melanogenesis-associated transcription factor (MITF), Myosin 5A (MYO5A), Premelanosome protein (PMEL), Melanophilin (MLPH), all genes related to the pigmentation process in the hair follicle and skin. Furthermore, in a hair follicle culture test, the inventors demonstrated that *Lacticaseibacillus paracasei* lysate was able to significantly increase melanin production in the hair bulb.

Thus, the application of the cosmetic, dermatological or pharmaceutical compositions of the invention promotes the prevention and/or reversal of the graying process of hair.

According to another of its aspects, the present invention refers to the use of a lysate of *Lacticaseibacillus paracasei* or a fraction thereof for the preparation of a pharmaceutical and/or dermatological composition, intended for the prevention and/or reversal of the graying process of hair.

In the context of the present invention, the term "prevent" means reducing the risk of manifestation of the phenomenon in question.

According to another aspect of the invention, it relates to a cosmetic method for treating and/or preventing the graying process, comprising at least one step of administering a lysate of *Lacticaseibacillus paracasei* or a fraction thereof to an individual in an amount effective for the treatment and/or prevention in question.

According to a further aspect of the present invention, said lysate is applied topically to a region comprising hair follicles.

### Microorganism(s)

As previously mentioned, the microorganism of the species *Lacticaseibacillus paracasei* , also known as *Lactobacillus paracasei* (previous taxonomic definition of the microorganism and updated as described by Zheng et al. 2020 in the new taxonomic classification of the genus Lactobacillus in the specialized journal " International Journal of Systematic and Evolutionary Microbiology*"*) , used as the active composition presented in the present patent application is used in the form of a lysate.

A lysate commonly denotes a material obtained after the destruction or dissolution of biological cells by a phenomenon called cell lysis, thus causing the release of the intracellular biological constituents naturally contained in the cells of the microorganism in question.

In the context of the present invention, the term lysate is used to denote the entire lysate obtained by lysis of the microorganism in question or only a fraction thereof. Thus, the invention relates to the application of a lysate of the species *Lacticaseibacillus paracasei* and/or a fraction thereof for the purposes specified herein.

The lysate used is therefore formed entirely or partially from the intracellular biological constituents and/or the constituents of the cell walls and membranes. This cell lysis can be carried out by various technologies, for example osmotic shock, heat shock, with ultrasound, or under mechanical stress such as centrifugation.

According to a preferred embodiment of the present invention, the lysate is obtained by cell lysis between 55 °C and 80 °C, with centrifugation being performed at the end of the process.

In one example, microorganisms of the species *Lacticaseibacillus paracasei* are grown anaerobically in a suitable culture medium. When the stationary phase of development is reached, the culture medium is inactivated and lysed, for example by heating to a temperature between 55°C and 80°C, optionally between 60°C and 65°C for 30 min by a pasteurization process. The microorganisms are then removed by a conventional separation technique, for example membrane filtration, centrifuged and subsequently the supernatant is used as product.

The active ingredient forming the lysate and belonging to the species *Lacticaseibacillus paracasei* may be formulated in a composition at a concentration of at least 0.0001% (expressed in dry weight), in particular at a concentration of 0.001 to 20% and more particularly at a concentration of 0.01 to 5% of the dry weight of the active substance relative to the total weight of the vehicle or of the composition containing it.

The compositions according to the invention may be presented in all galenic forms normally available for the method of administration adopted.

The carrier/vehicle can be of a varied nature depending on the type of composition considered.

More particularly with regard to compositions intended for topical administration, these may be an aqueous, aqueous-alcoholic or oily solution, a solution-type dispersion or lotion-type or serum-type dispersion, an emulsion of liquid or semi-liquid consistency, obtained by dispersing an oily phase in an aqueous phase (O/W) or vice versa (W/O), or a suspension or emulsion of soft, semi-solid or solid consistency, of the cream, aqueous or anhydrous gel type, or microemulsions, microcapsules, microparticles or vesicular dispersions of the ionic and/or non-ionic type.

In one embodiment of the present invention, the product formulation may be Benzyl alcohol: 0.80%; Ethylhexylglycerin: 0.20% and Lacticaseibacillus paracasei lysate: qsp 100%.

These compositions may notably constitute creams for cleansing, protecting, treating or caring for the scalp, lotions, gels or mousses for the care of the scalp, such as cleansing or disinfecting lotions or bath compositions. They may also be used for the scalp in the form of solutions, creams, gels, emulsions, mousses or in the form of aerosol compositions also containing a propellant under pressure. Specifically, they may be in the form of hair tonics, conditioners, leave-on products, shampoos, among others.

The aforementioned ingredient with anti-gray action has characteristics that allow it to be adapted to different types of products with different compositions for hair care, such as:

Alcohol-free hair tonic, by means of compositions comprising 0.10% xylityl sesquicaprylate, 2% PPG-5-Ceteth 20, 10% *Lacticaseibacillus paracasei lysate* and water qsp 100%.

Hair tonic with alcohol, by means of compositions comprising 0.10% xylityl sesquicaprylate, 20% ethyl alcohol 96%, 2% PPG-5-Ceteth 20, 10% *Lacticaseibacillus paracasei* lysate and water qsp 100%.

Conditioner, by means of compositions comprising 0.10% disodium EDTA, 1% glycerin, 0.50% cetyl trimethyl ammonium chloride, 0.22% lactic acid 85%, 3.00% cetostearyl alcohol 30/70, 1.00% cetyl alcohol, 0.20% stearamidopropyl dimethyl amine, 0.50% xylityl sesquicaprylate, 2% *Lacticaseibacillus paracasei lysate* and water qsp 100%.

*Leave on,* through compositions comprising 0.10% disodium EDTA, 1% glycerin, 0.80% cetyl trimethyl ammonium chloride, 0.10% citric acid 50%, 0.22% lactic acid 85%, 3.00% cetostearyl alcohol 30/70, 0.70% cetyl alcohol, 0.30% eyoxylated cetostearyl alcohol 20 EO, 0.50% xylityl sesquicaprylate, 2% Lacticaseibacillus paracasei lysate and water qsp 100%.

Shampoo, by means of compositions comprising 4% cocamidopropyl betaine, 27% sodium lauryl ether sulfate, 3% sodium lauroyl sarcosinate, 1% laurel glucoside, 4% coccoamide DEA, 0.50% xylityl sesquicaprylate, sodium chloride qsp, 50% citric acid solution qsp pH 6.00 - 7.00, *2% Lacticaseibacillus paracasei lysate* and water qsp 100%.

As is known, galenic forms intended for topical administration may also contain additives commonly used in the cosmetic, pharmaceutical and/or dermatological fields, such as hydrophilic or lipophilic gelling agents, hydrophilic or lipophilic active ingredients, preservatives, antioxidants, solvents, perfumes, fillers, filters, odor absorbers and colorants. The quantities of these various additives are those classically used in the field in question, for example from 0.01 to 20% of the total weight of the composition. Depending on their nature, these additives may be introduced in the fatty/oily phase and/or in the aqueous phase.

The products may be used as cosmetic, dermatological or pharmaceutical compositions for topical use capable of modulating the production of melanin in the hair follicle. These compositions may in particular constitute creams for cleansing, protecting, treating or caring, lotions, gels , shampoos, conditioners or mousses for the care of the scalp, such as cleansing or disinfecting lotions or bath compositions.

They can also be used for the scalp in the form of solutions, creams, gels, emulsions, mousses or in the form of aerosol compositions also containing a propellant under pressure.

### Example 1: Assessment of gene expression modulation in human skin fragments

### Material and methods

Skin explants (n=6), obtained from a surgical procedure performed by an ophthalmological plastic surgery clinic, were subjected to 0.4 cm² cut. The obtained fragments were transferred to a cell culture plate containing a culture medium for treatment with a formulation containing *Lacticaseibacillus paracasei* lysate (3% w/w) and the same formulation without the addition of the lysate (placebo). After treatment, the fragments were kept in a humid environment, 37 °C, 5% CO₂ for 24 hours, and were subsequently collected in RNAlaterTM solution (Qiagen).

After 24 hours of incubation with the formulations and storage in RNAlaterTM solution (Qiagen), the *ex vivo tissue* was mechanically macerated and then the RNA was extracted with a commercially available kit, quantified, and analyzed for its integrity.

cDNA synthesis was performed with the High-Capacity cDNA Reverse Transcription^{®} kit (Applied Biosystems) and conducted on the StepOnePlus device (Applied Biosystems). Subsequently, cDNA was quantified on a Nanodrop Lite device (Thermo Scientific)

High-throughput RT-qPCR was performed using the Biomark^{™} HD System (Fluidigm, San Francisco, CA) associated with the IFC Controller HX, which supports the IFC 96.96 Dynamic Array^{™}. Finally, the cycle threshold was determined specifically for each assay using Fluidigm Software and the expression results were analyzed using the delta-delta Ct method.

### Results

*Lacticaseibacillus paracasei* lysate (3% w/w) was performed in comparison to the expression of the same genes from skin fragments treated only with a formulation without the lysate (placebo).

*Lacticaseibacillus paracasei* lysate, in relation to the placebo group.

| **Modulation (%) of gene expression in the treatment of skin with the composition of the present invention vs. placebo** | | | | | |
|---|---|---|---|---|---|
| **TYR** | **TRP-2/DCT** | **TYRP-1** | **MITF** | **MYO5A** | **PMEL** |
| +147% | +140% | +113% | +109% | +104% | +86% |

| **MLPH** | **KIT** | **LEF1** | **POMC** | **MC1R** | |
|---|---|---|---|---|---|
| +62% | +100% | +90% | -30% | -26% | |

| | | | | | |
|---|---|---|---|---|---|
| Table 1. Percentage of modulation of relative gene expression in skin fragments treated with formulation containing *Lacticaseibacillus paracasei lysate* (3%), in relation to the placebo group. | | | | | |

### Example 2: Evaluation of the induction of melanin synthesis in follicle culture

### Material and methods

Hair follicles were dissected and seeded in 24-well plates (1 follicle/well) in culture medium containing or not (control) *Lacticaseibacillus paracasei lysate* (0.002% w/w) and a comparison composition (*benchmark =* Glycerin, Water and Palmitoyl Tetrapeptide-20 Amide - 0.002% w/w). For each test condition, an excess of hair follicles was prepared to reach the target value of 12 high-quality hair follicles per condition at the end of the experiment. Hair follicles were incubated for 7 days with renewal of the treatment by removing and replacing the medium after 3 and 5 days of incubation.

At the end of the incubation, the hair follicles were fixed with formaldehyde solution. The follicles were then dehydrated and then embedded in paraffin. Cross sections were made with a microtome (5 µm thick).

The sections were deparaffinized and then subjected to the Warthin Starry staining method. Finally, the sections were observed under a microscope (40x objectives) and images were recorded. The amount of melanin was assessed by measuring the marked area of melanin in the bulb normalized by the area of the bulb (semiquantitative image analysis method).

### Results

*Lacticaseibacillus paracasei* lysate increased melanin levels by 43% compared to the control group (without treatment). The comparator product, under the same conditions, increased melanin levels in the follicles by 14% (Figure 1).

*Lacticaseibacillus paracasei* lysate was able to intensely stimulate melanin synthesis in the hair follicle bulb. The quantification of the pigmented area indicates an increase of 43% in relation to the control group, while the *benchmark* was able to stimulate pigmentation by 14%, under the same experimental conditions.

The examples disclosed herein are intended to merely exemplify some of the numerous forms of realization and use of the present invention, without limiting the interpretation of its scope and breadth, as well as possible alternative forms and configurational variations thereof that will be defined based on the claims presented herein.

## Claims

1. Cosmetic composition for inducing melanin synthesis in an individual or animal **characterized by** comprising at least 0.0001% by weight of lysate of microorganisms of the species *Lacticaseibacillus paracasei.*

2. Composition according to claim 1, **characterized in that** it comprises between 0.001% and 20% by weight of lysate.

3. Composition according to claim 1, **characterized in that** it comprises between 0.001 % and 5% by weight of lysate.

4. Composition, according to claim 1, 2 or 3, **characterized by** being for preventing and/or reversing the graying process of hair strands.

5. Composition according to any one of the preceding claims, **characterized in that** the induction of melanin synthesis is in hair follicles.

6. Composition according to any one of the preceding claims, **characterized in that** it is a topical cosmetic composition.

7. Process for producing a composition, as defined in any of the preceding claims, **characterized by** comprising:
- anaerobic cultivation of microorganisms of the species *Lacticaseibacillus paracasei* in culture medium until the stationary phase;
- total or partial inactivation and lysis of microorganisms by subjecting the medium to a temperature between 55°C and 80°C for at least 30 minutes;
- filtration and centrifugation;
- mixing the resulting lysate with at least one suitable cosmetic vehicle.

8. Cosmetic method of inducing melanin synthesis in an individual or animal, **characterized by** comprising the topical administration of a composition as defined in any one of claims 1 to 6, in a cosmetically suitable amount to allow said induction in an individual or animal.

9. Method, according to claim 8, **characterized in that** it is for the prevention and/or reversal of the graying process of hair strands and said topical administration is on the hair follicles.

10. Use of the composition, as defined in any one of claims 1 to 6, **characterized in that** it is for the production of a cosmetic composition for the induction of melanin synthesis in an individual or animal.

11. Use according to claim 10, **characterized in that** the composition is for preventing and/or reversing the graying process of hair strands and for application to a region of skin comprising hair follicles.
